# DEMANDE DE BREVET EUROPEEN

(11) **EP 2 272 506 A2**
(43) Date de publication de la demande: **12.01.2011**
(21) Numéro de dépôt: 10184459.5
(22) Date de dépôt: 28.07.2003
(51) Int. Cl.: A61K 9/50

(54) **Microcapsules a liberation modifiee de principes actifs peu solubles pour administration per os**

(30) Priorité: 26.07.2002 FR 0209530
(62) Demande divisionnaire de: 03750853.8
(71) Demandeur: Flamel Technologies, 69200 Venissieux (FR)
(72) Inventeur: Guimberteau, Florence, 33450, Montussan (FR); Castan, Catherine, 69530, Orlienas (FR); Meyrueix, Rémi, 69009, Lyon (FR); Soula, Gérard, 69330, Meyzieu (FR)
(74) Mandataire: Cabinet Plasseraud

(57) **Abrégé**

La présente invention concerne des microcapsules permettant la libération modifiée de façon fiable et industriellement reproductible d'un principe (PA) peu soluble dans l'eau, *à l'exclusion des anti-hyperglycémiants.* Chacune de ces microcapsules comprend un coeur de PA peu soluble et une pellicule d'enrobage appliquée sur le coeur . Leur diamètre moyen est inférieur à 1000 microns. La pellicule d'enrobage contient un polymère filmogène (P1) insoluble dans les liquides du tractus gastro-intestinal; un polymère hydrosoluble (P2); un plastifiant (PL); et éventuellement un agent tensioactif (TA) lubrifiant. Cette pellicule d'enrobage représente au moins 3 à 40% p/p sec de leur masse totale, et ses composants P1, P2, PL satisfont aux caractéristiques suivantes : fraction massique en poids sec de P1 par rapport à la masse totale de l'enrobage, comprise entre 50 et 80%; fraction massique en poids sec P2/P1+P2 comprise entre 15 et 60 %; fraction massique en poids sec PL/P1+PL comprise entre 1 et 30 %.

L'invention concerne aussi les applications desdites microcapsules et desdits enrobages en galénique.

## Description

Le domaine de la présente invention est celui des systèmes à libération modifiée de principes actifs médicamenteux et/ou nutritionnels (PA), destinés à une administration par la voie orale.
La présente invention concerne ainsi des microcapsules destinées à l'administration *per os* et contenant au moins un PA peu soluble.
L'invention concerne également les médicaments contenant ces microcapsules évoquées ci-dessus et l'utilisation de ces dernières pour la fabrication de médicaments.

Dans le présent exposé, l'expression"*libération modifiée*", désigne indifféremment une libération du (ou des) principe(s) actif(s) débutant dès la mise en contact de la forme galénique avec son milieu de dissolution (in vivo ou in vitro) ou bien encore une libération du (ou des) principe(s) actif(s) ne débutant qu'après une durée prédéterminée allant par exemple de 0,5 à plusieurs heures. Ainsi au sens de l'invention, une prolongation de la libération correspond à un temps de libération de 50% du (ou des) principe(s) actif(s) qui est typiquement de plusieurs heures et qui peut s'étendre de 0,25 à 20 heures, par exemple.
L'expression "*faible solubilité*" se rapporte à des principes actifs dont la solubilité dans l'eau est inférieure à 10 g/l à 25°C.

Plus précisément, l'invention concerne des formulations pharmaceutiques à libération prolongée de principes actifs de faible solubilité, cette formulation étant constituée d'une pluralité de microcapsules constituées d'un coeur contenant le principe actif de faible solubilité et enrobé d'une couche de polymère qui contrôle la libération du PA.

Parmi les différents systèmes à libération modifiée, les systèmes galéniques à libération modifiée constitués d'une pluralité de microcapsules de type réservoir de diamètre moyen inférieur à 1000 microns, sont particulièrement avantageux. En effet, dans ces systèmes, la dose de principe(s) actif(s) à administrer se répartit entre grand nombre de microcapsules (typiquement 10 000 pour une dose de 500 mg et un diamètre de 400 microns) et ce type de système présente, de ce fait, les avantages intrinsèques suivants :
- La mise en oeuvre d'un mélange de microcapsules de profils de libération modifiée différents, permet de réaliser des profils de libération présentant plusieurs vagues de libération ou assurant par un réglage adéquat des différentes fractions, un niveau de concentration plasmatique du PA constant;
- la sensibilité à la variabilité de la vidange gastrique est moindre, car la vidange, qui s'effectue ici sur un grand nombre de particules est statistiquement plus reproductible;
- On évite la mise en contact des tissus avec une dose élevée en PA « dose dumping ». Chaque microcapsule ne contient en effet qu'une dose très réduite en principe(s) actif(s). On s'affranchit ainsi du risque de détérioration des tissus par surconcentration locale de principe(s) actif(s) agressif;
- Il est possible de combiner plusieurs formes galéniques (libération immédiate et/ou retardée et/ou prolongée) comportant un ou plusieurs principes actifs, dans ces systèmes "multimicrocapsulaires";
- il n'induit pas de dégradation du PA;
- Le temps de séjour des microcapsules dans les parties hautes du tractus peut être prolongé, ce qui assure un accroissement de la durée de passage du (ou des) principe(s) actif(s) devant les fenêtres d'absorption et maximise ainsi la biodisponibilité du (ou des) principe(s) actif(s).

Dans le cas cependant où la solubilité du PA est faible, la réalisation d'une forme microparticulaire à libération modifiée se heurte à une difficulté importante.

La diffusion du principe actif à travers la pellicule d'enrobage entourant chaque microcapsule s'effectue sous l'action du gradient de concentration en PA dissous entre l'intérieur et l'extérieur de la microcapsule. En d'autres termes, c'est la différence de pression osmotique du PA entre l'intérieur et l'extérieur de la microcapsule qui est le moteur de la libération. La concentration interne en PA est la concentration à saturation. La concentration externe en PA est quant à elle négligeable, en conditions usuelles (dites " sink "). Le moteur de la libération est donc directement lié à la concentration à saturation du PA, c'est à dire à sa solubilité.
Pour les PA peu solubles, la concentration à saturation en PA est peu élevée et la diffusion du PA vers l'extérieur est donc a priori très lente, même pour des pellicules d'enrobage peu épaisses .
Et de toutes façons, pour des pellicules d'enrobage de faibles épaisseurs, on se heurte alors aux difficultés suivantes :
(a) Le dépôt d'une pellicule d'enrobage de très faible épaisseur n'est pas régulier : des lacunes côtoient des zones de surépaisseurs, et la libération du PA n'est pas prolongée.
(b) Le contrôle industriel du procédé de dépôt d'une très faible épaisseur devient très délicat et peu reproductible.

Par ailleurs, pour les pellicules d'enrobage plus épaisses, la libération du PA est extrêmement lente, voire inexistante.

Ce problème technique est d'autant plus délicat à résoudre que cela ne doit pas se faire au détriment des autres spécifications requises pour un système galénique pour l'administration de PA par voie orale, qui sont entre autres, cumulativement et pour une large gamme de PA, les suivantes :
- transit lent dans les parties hautes du tractus gastro-intestinal, reflété par un profil d'absorption in vivo sur une durée notablement supérieure à celle permise par le transit naturel (3 h +/- 1),
- absence d'irritation de la muqueuse,
- masse limitée de la forme galénique correspondant à une dose,
- coût de revient réduit.

La difficulté à modifier la libération d'un PA peu soluble explique le petit nombre de solutions techniques qui ont été proposées à ce jour.

S'agissant des systèmes galéniques, solides, multimicrocapsulaires, on connaît ceux constitués par une multiplicité de particules ou microcapsules portant chacune du principe(s) actif(s) enrobé d'une couche de pelliculage à base d'éthylcellulose, de polyvinylpyrrolidone, de stéarate de magnésium et d'huile de ricin, par exemple. Un tel système galénique est divulgué dans la demande PCT WO-96/11675. Ces microcapsules-réservoirs tirent de leur multiplicité un avantage, qui est un temps de vidange gastrique plus régulier et reproductible. De plus, leur taille comprise entre 50 et 1 000 µm ainsi que les caractéristiques de leur enrobage permet d'accroître leur temps de transit dans les parties hautes du tractus gastro-intestinal et, par suite, de maintenir l'absorption du principe(s) actif(s) pendant tout ou partie de ce temps de séjour dans l'intestin grêle.
Mais, le système galénique multimicrocapsulaire selon le WO-96/11675 est perfectible en ce qui concerne les PA peu solubles administrables oralement, car il ne propose pas de solution au problème de la diffusion d'un tel PA peu soluble à travers une pellicule d'enrobage d'une épaisseur suffisamment importante, par exemple de plusieurs microns.

Dans le domaine des microcapsules à libération modifiée de principes actifs anti-hyperglycémiants, il convient de mentionner la demande de brevet français FR-A-2 816 840 qui divulgue des microcapsules dont le coeur est constitué par des cristaux de metformine, enrobés par une membrane de contrôle de la libération de la metformine comprenant de l'acide stéarique (50%) ou de l'huile de ricin (10%) et de l'éthylcellulose (respectivement 50 et 90 %). Ce système galénique, pour l'administration orale de principes actifs anti-hyperglycémiques, doit permettre d'obtenir une couverture thérapeutique performante sur 24 heures en surmontant les problèmes de bipasse de la fenêtre d'absorption et de libération massive localisée de principe actif.
Cette proposition technique reste perfectible, dans la mesure où elle ne résout pas le problème des PA faiblement solubles évoqué ci-dessus

S'agissant de l'art antérieur sur les microcapsules à libération modifiée de principe actifs peu solubles, il convient de mentionner tout d'abord la demande de brevet PCT WO-99/49846 qui décrit une préparation pharmaceutique composée de particules submicroniques (0,05 à 10 µm) associant un principe actif peu soluble avec un composé phospholipidique, un composé modifiant les charges de surface et un polymère bloc. Cette préparation a pour objectif d'améliorer la biodisponibilité et la stabilité du principe actif et trouve ses applications dans des formes injectables ou bien destinées à être administrées par voie oculaire ou nasale. Une forme à libération prolongée n'est obtenue qu'en cas d'injection intramusculaire.
La demande de brevet PCT WO-00/18374 décrit une invention de même nature que la précédente : le principe actif sous forme de particules submicroniques (< 1000 nm) est stabilisé par un composé associé à la surface des particules et mélangé à un polymère. Ce mélange peut ensuite être mis sous forme de granules ou pellets et éventuellement de comprimé. Le principe actif est rapidement dissous et c'est l'augmentation de la biodisponibilité obtenue grâce à la réduction de taille qui permet d'avoir une concentration plasmatique efficace sur une période prolongée.
La demande de brevet GB-2 202 143 décrit des sphéroïdes de diamètre supérieur à 0,5 mm et de préférence supérieur à 0,8 mm, contenant le principe actif faiblement soluble dispersé dans 70 à 99,5% de cellulose microcristalline. Cette forme matricielle ne requiert aucun enrobage contrôlant la libération du principe actif.
La demande de brevet JP-8073345 décrit un système à libération contrôlée composé d'un granulé pelliculé. Le granulé contient un principe actif faiblement soluble à pH neutre et des acides inorganiques. Ce système propose donc une solution adaptée uniquement au cas des principes actifs basiques faiblement solubles.
Enfin, le brevet européen EP-B-0 249 587 concerne une préparation solide permettant la libération lente d'une substance active, faiblement soluble (< 0,1 % en poids). Cette préparation à libération contrôlée peut se présenter sous forme de gélules comprenant des capsules constituées de granules enrobés. Les granules comprennent le principe actif peu soluble et un solubilisant constitué par le produit commercial Cremophor® RH 40 (huile de ricin hydrogénée polyoxyéthylénée : 40 motifs oxyde d'éthylène), ainsi que d'autres additifs tels que la polyvinylpyrrolidone, la cellulose, l'amidon et le lactose. Ces granules de taille comprise entre 700 et 1120 µm sont revêtus d'une couche d'enrobage en éthylcellulose, permettant le contrôle de la libération. Les ingrédients des granules que sont la polyvinylpyrrolidone, la cellulose, l'amidon de mais et le lactose, semblent être les éléments du système de gel hydrophile propre à la forme galénique selon l'EP-B-0 249 587. Ces capsules comportent donc un seul constituant (éthylcellulose) dans leur couche d'enrobage, ce qui limite ses capacités en matière de modification de la libération du principe actif. En particulier, il est douteux qu'une couche d'enrobage uniquement composée d'éthylcellulose (connue pour former des films imperméables), permette la libération d'un PA peu soluble, de manière contrôlée et industriellement reproductible, sur une durée de quelques heures, par exemple.

Aucune de ces demandes de brevet ne décrit des microparticules de type réservoir ou microcapsules pour lesquelles la libération prolongée du principe actif peu soluble est contrôlée par sa diffusion au travers d'une membrane ayant une épaisseur suffisante pour assurer une perméabilité contrôlée et industriellement reproductible.-Elles n'enseignent pas non plus de quelle façon aboutir à un tel système.

Devant cette vacuité de l'art antérieur, l'un des objectifs essentiels de la présente invention est de proposer une forme à libération modifiée de PA peu soluble(s) constituée d'une pluralité de microcapsules, formée chacune par un coeur contenant le PA et enrobé d'une pellicule d'enrobage.
Un autre objectif de la présente invention est de fournir une pluralité de microcapsules de type réservoir de PA de faible solubilité, pour l'administration orale de ce (ou ces) dernier(s), la pellicule d'enrobage de ces microcapsules ayant une épaisseur suffisante pour assurer une perméabilité contrôlée et industriellement reproductible.
Un autre objectif essentiel de la présente invention est de fournir une pluralité de microcapsules de PA peu soluble(s), de taille inférieure à 1000 µm.
Un autre objectif de la présente invention est de proposer une forme galénique orale et constituée d'un grand nombre (par exemple de l'ordre de plusieurs milliers) de microcapsules, cette multiplicité assurant statistique-ment une bonne reproductibilité de la cinétique de transit du PA dans tout le tractus gastro-intestinal, de sorte qu'il en résulte un meilleur contrôle de la biodisponibilité et donc une meilleure efficacité.
Un autre objectif essentiel de la présente invention est de fournir une pluralité de microcapsules de PA peu soluble(s), pour l'administration orale de ce (ou ces) dernier(s) selon un profil de libération prolongée et/ou éventuellement retardée, de telle sorte que le temps de demi-libération t_{1/2} soit compris entre 0,25 et 20 heures.
Un autre objectif essentiel de la présente invention est de fournir une forme orale à libération modifiée dans laquelle le (ou les) PA est (sont) sous forme de pluralité de particules enrobées individuellement pour former des microcapsules et dans laquelle il est possible de mélanger plusieurs principes actifs sous forme multimicrocapsulaire, libérés selon des temps de libération respectifs différents.

S'étant fixés tous les objectifs ci-dessus parmi d'autres, les inventeurs ont eu le mérite de mettre au point un système galénique multimicrocapsulaire à libération prolongée de PA peu soluble, par voie orale, qui, outre les propriétés visées dans les objectifs ci-dessus, possède, cumulativement et pour une large gamme de PA, les spécifications suivantes entre autres :
- absence d'irritation de la muqueuse,
- teneur en PA élevée,
- coût de revient réduit,
- qui permette d'ajuster le temps de demi-libération du PA entre 0,25 et 20 heures,
- qui soit reproductible et aisé à mettre en oeuvre industriellement grâce à un rapport de la masse de la pellicule d'enrobage à la masse de la particule supérieur à 3 % p/p sec, de préférence supérieur à 5 % p/p sec,et, plus préférentiellement encore compris entre 3 et 40 % p/p sec.

Pour ce faire, les inventeurs ont eu le mérite de découvrir après de nombreux essais des microcapsules de structure particulière qui permettent de satisfaire aux objectifs rappelés ci dessus, parmi d'autres.

A cette fin, la présente invention a pour objet un système galénique formé par des microcapsules permettant la libération modifiée d'au moins un PA peu soluble dans l'eau, *à l'exclusion ou non des anti-hyperglycémiants*, destinées à être administrées par voie orale et du type de celles :
- constituées chacune par un coeur comportant au moins un principe actif et par une pellicule d'enrobage appliquée sur le coeur et régissant la libération modifiée du (ou des) PA,
- dont le diamètre moyen est inférieur à 1000 microns, de préférence compris entre 800 et 50 microns et plus préférentiellement encore compris entre 600 et 100 microns,
- dont la pellicule d'enrobage de chaque microcapsule contient les composants suivants :
   → -I-- au moins un polymère filmogène (P1) insoluble dans les liquides du tractus gastro-intestinal ;
   → -II-- au moins un polymère hydrosoluble (P2) ;
   → -III- au moins un plastifiant (PL) ;
   → -IV- et éventuellement au moins un agent tensioactif (TA) lubrifiant ;
*à l'exclusion ou non des pellicules d'enrobage constituées par des compositions entériques et des pellicules d'enrobage de composition ci-après:*
*1 - au moins un polymère filmogène (P1) insoluble dans les liquides du tractus, présent à raison de 50 à 90, de préférence 50 à 80 % en poids sur sec par rapport à la masse totale de la composition d'enrobage et constitué par au moins un dérivé non hydrosoluble de la cellulose, à savoir l'éthylcellulose et*/*ou l'acétate de cellulose ;*
*2 - au moins un polymère azoté (P2) présent à raison de 2 à 25, de préférence 5 à 15 % en poids sur sec par rapport à la masse totale de la composition d'enrobage et constitué par au moins un polyacrylamide et*/*ou un poly-N-vinylamide et*/*ou un poly-N-vinyl-lactame, à savoir le polyacrylamide et*/*ou la polyvinylpyrrolidone ;*
*3 - au moins un plastifiant présent à raison de 2 à 20, de préférence de 4 à 15 % en poids sur sec par rapport à la masse totale de la composition d'enrobage et constitué par au moins l'un des composés suivants : les esters du glycérol, les phtalates, les citrates, les sébaçates, les esters de l'alcool cétylique, l'huile de ricin, l'acide salicylique et la cutine ;*
*4 - et au moins un agent tensioactif et*/*ou lubrifiant, présent à raison de 2 à 20, de préférence de 4 à 15 % en poids sur sec par rapport à la masse totale de la composition d'enrobage et choisi parmi les tensioactifs anioniques, à savoir les sels alcalins ou alcalinoterreux des acides gras, l'acide stéarique et*/*ou oléique étant préférés, et*/*ou parmi les tensioactifs non ioniques, à savoir les esters de sorbitan polyoxyéthylénés et*/*ou les dérivés de l'huile de ricin polyoxyéthylénés, et*/*ou parmi les agents lubrifiants comme les stéarates de calcium, de magnésium, d'aluminium ou de zinc, ou comme le stéarylfumarate de sodium et*/*ou le béhénate de glycérol; ledit agent pouvant comprendre un seul ou un mélange des susdits produits*;
**caractérisées**:
➢ **en ce que** leur pellicule d'enrobage représente au moins 3 % p/p sec, de préférence au moins 5% p/p sec de leur masse totale,
➢ et en ce que les composants P1, P2, PL de la pellicule d'enrobage satisfont aux caractéristiques suivantes :
   ➢ fraction massique en poids sec de P1 par rapport à la masse totale de l'enrobage, comprise entre 40 et 90% et de préférence entre 50 et 80%;
   ➢ fraction massique en poids sec P2/P1+P2 comprise entre 15 et 60 % et de préférence entre 15 et 55%;
   ➢ fraction massique en poids sec PL/P1+PL comprise entre 1 et 30 % et de préférence entre 5 et 25 %.

II est du mérite de la Demanderesse, d'avoir mis au point de façon tout à fait surprenante et inattendue, un tel système galénique permettant une diffusion du PA peu soluble au travers d'une pellicule d'enrobage des microcapsules suffisamment épaisse et sans grever le coût de revient.
Le choix d'un taux de pelliculage supérieur ou égal à 3 % en poids sur sec par rapport à la masse totale de la microcapsule, est une disposition particulièrement inventive, qui s'inscrit à contre courant de l'opinion technique généralement répandue dans ce domaine.
Il en va de même s'agissant des données quantitatives pour P1, P2 & PL.

Suivant un mode particulièrement préféré de réalisation de l'invention, la pellicule d'enrobage représente 3 à 40 % p/p sur sec de la masse totale des microcapsules.

De préférence, P1 est sélectionné dans le groupe de produits suivants :
- les dérivés non hydrosolubles de la cellulose, de préférence l'éthylcellulose et/ou l'acétate de cellulose,
- les dérivés acryliques,
- les polyvinylacétates,
- et leurs mélanges.

De préférence, P2 est sélectionné dans le groupe de produits suivants :
- les dérivés hydrosolubles de la cellulose,
- les polyacrylamides,
- les poly-N-vinylamides,
- les poly-N-vinyl-lactames,
- les alcools polyvinyliques (APV),
- les polyoxyéthylènes (POE),
- les polyvinylpyrrolidones (PVP) (ces dernières étant préférées),
- et leurs mélanges.

De préférence, PL est sélectionné dans le groupe de produits suivants :
- le glycérol et ses esters, de préférence dans le sous-groupe suivant: glycérides acétylés, glycérolmono-stéarate, glycéryltriacétate, glycéroltributyrate,
- les phtalates, de préférence dans le sous-groupe suivant: dibutylphthalate, diéthylphthalate, diméthylphthalate, dioctylphthalate,
- les citrates, de préférence dans le sous-groupe suivant : acétyltributylcitrate, acétyltriéthylcitrate, tributylcitrate, triéthylcitrate,
- les sébaçates, de préférence dans le sous-groupe suivant: diéthylsébaçate, dibutylsébaçate,
- les adipates,
- les azélates,
- les benzoates,
- les huiles végétales,
- les fumarates de préférence le diéthylfumarate,
- les malates, de préférence le diéthylmalate,
- les oxalates, de préférence le diéthyloxalate,
- les succinates; de préférence le dibutylsuccinate,
- les butyrates,
- les esters de l'alcool cétylique,
- l'acide salicylique,
- la triacétine,
- les malonates, de préférence le diéthylmalonate,
- la cutine,
- l'huile de ricin (celle-ci étant particulièrement préférée),
- et leurs mélanges.

Selon une variante avantageuse, la pellicule d'enrobage comprend du composant TA à raison de 2 et 20 % et de préférence entre 4 et 15 % de la masse totale de l'enrobage sec.

De préférence, TA est sélectionné dans le groupe de produits suivants :
- les tensioactifs anioniques, de préférence dans le sous-groupe des sels alcalins ou alcalinoterreux des acides gras, l'acide stéarique et/ou oléique étant préférés,
- et/ou les tensioactifs non ioniques, de préférence dans le sous-groupe suivant:
   ○ les huiles polyoxyéthylénées de préférence l'huile de ricin hydrogénée polyoxyéthylénée,
   ○ les copolymères polyoxyéthylène-polyoxypropylène,
   ○ les esters de sorbitan polyoxyéthylénés,
   ○ les dérivés de l'huile de ricin polyoxyéthylénés,
   ○ les stéarates, de préférence de calcium, de magnésium, d'aluminium ou de zinc,
   ○ les stéarylfumarates, de préférence de sodium,
   ○ le béhénate de glycérol,
   ○ et leurs mélanges.

Avantageusement, les microcapsules sont conçues de manière à pouvoir séjourner dans les parties hautes du tractus gastro-intestinal pendant d'au moins 5 heures environ, de préférence au moins 8 heures environ, et permettre ainsi l'absorption du PA pendant une durée prolongée.

Suivant un mode particulier de réalisation des microcapsules à PA peu solubles selon l'invention et selon un autre mode d'expression quantitatif, la pellicule d'enrobage comprend de 35 à 75 % d'éthylcellulose P1, de 20 à 50 % de polyvinylpyrrolidone P2, de 5 à 15 % de PL.

Cette préparation selon l'invention permet de réaliser une forme multimicrocapsulaire à libération modifiée de PA peu solubles, le temps de demi-libération du PA pouvant être ajusté entre 0,25 et 20 heures de façon reproductible grâce à l'utilisation d'une pellicule d'enrobage que l'on peut qualifier de pellicule d'enrobage de diffusion, suffisamment épaisse.

Par ailleurs, pour des PA peu solubles dont la fenêtre d'absorption est limitée, une telle pluralité de microcapsules (typiquement 10 000 pour une dose de 500 mg et un diamètre moyen de 400 microns) présente les avantages intrinsèques suivants :
- La mise en oeuvre d'un mélange de microcapsules de profils de libération retardée et contrôlée différents, permet de réaliser des profils de libération présentant plusieurs vagues de libération ou assurant par un réglage adéquat des différentes fractions, un niveau de concentration plasmatique du PA constant.
- La variabilité de la vidange gastrique est moindre, car la vidange qui s'effectue ici sur un grand nombre de particules est statistiquement plus reproductible.
- On évite la mise en contact des tissus avec une dose élevée en PA "*dose dumping*". Chaque microcapsule ne contient en effet qu'une dose très réduite en PA. On s'affranchit ainsi du risque de détérioration des tissus par surconcentration locale de PA agressif.
- Le temps de séjour des microcapsules dans les parties hautes du tractus peut être prolongé, ce qui assure un accroissement de la durée de passage du PA devant les fenêtres d'absorption et maximise ainsi la biodisponibilité du PA.

Les PA peu solubles utilisés pour la préparation des microcapsules à libération modifiée, de préférence contrôlée, selon l'invention peuvent être choisis parmi au moins l'une des grandes variétés de substances actives suivantes :
antiulcéreux, antidiabétiques, anticoagulants, antithrombiques, hypo-lipémiants, antiarythmiques, vasodilatateurs, antiangineux, antihypertenseurs, vasoprotecteurs, promoteurs de fécondité, inducteurs et inhibiteurs du travail utérin, contraceptifs, antibiotiques, antifongiques, antiviraux, anticancéreux, anti-inflammatoires, analgésiques, antiépi-leptiques, antiparkinsoniens, neuroleptiques, hypnotiques, anxiolytiques, psychostimulants, anti-migraineux, antidépresseurs, antitussifs, antihista-miniques ou antiallergiques.

De préférence, le ou les PA est (sont) choisi(s) parmi les composés suivants : prazosine, acyclovir, nifedipine, naproxen, ibuprofen, ketoprofen, fenoprofen, indométhacine, diclofenac, sulpiride, terfenadine, carbamazepine, fluoxétine, alprazolam, famotidine, ganciclovir, spironolactone, acide acétylsalycilique, quinidine, morphine, amoxicilline, paracétamol, métoclopramide, vérapamil et leurs mélanges.

Selon une variante, le PA est constitué par au moins un supplément nutritionnel et/ou diététique, de préférence choisi parmi les vitamines, les acides aminés, les oligoéléments, les antioxydants et leurs mélanges.

S'agissant de la préparation des microcapsules selon l'invention, cela renvoie à des techniques de microencapsulation accessibles à l'homme du métier, dont les principales sont résumées dans l'article de C. DUVERNEY et J. P. BENOIT dans "L'actualité chimique", décembre 1986. Plus précisément, la technique considérée est la microencapsulation par pelliculage, conduisant à des systèmes "réservoir" individualisés par opposition aux systèmes matriciels.
Pour plus de détails, on se réfèrera au brevet EP-B-0 953 359.

Les particules de PA de granulométrie désirée et nécessaire à la réalisation des microcapsules selon l'invention peuvent être des cristaux de PA pur et/ou ayant subi un prétraitement par l'une des techniques conventionnelles en la matière, comme par exemple la granulation, en présence d'au moins un agent liant classique et/ou d'un agent modifiant les caractéristiques de solubilité intrinsèque du PA.

La présente invention vise également un médicament comprenant les microcapsules telles que définies ci-dessus.
Ce médicament peut être sous forme solide : comprimé, gélule, poudre, etc ou sous forme liquide, par exemple suspension aqueuse.

Conformément à l'invention, il est également proposé à titre de solution aux problèmes évoqués au début du présent exposé, à savoir : libération modifiée, de préférence prolongée, de PA peu solubles, dans une forme galénique avalable aisément, le tout dans une perspective de couverture thérapeutique longue, efficace et sûre, d'utiliser une pluralité de microcapsules permettant la libération modifiée d'au moins un PA peu soluble dans l'eau, *à l'exclusion ou non des anti-hyperglycémiants*, destinées à être administrées par voie orale, ces microcapsules présentant les caractéristiques suivantes :
- elles constituées chacune par un coeur comportant au moins un principe actif et par une pellicule d'enrobage appliquée sur le coeur et régissant la libération prolongée du (ou des) PA,
- leur diamètre moyen est inférieur à 1000 microns, de préférence compris entre 800 et 50 microns et plus préférentiellement encore compris entre 600 et 100 microns,
- leur pellicule d'enrobage contient les composants suivants :
   → -I-- au moins un polymère filmogène (P1) insoluble dans les liquides du tractus gastro-intestinal ;
   → -II-- au moins un polymère hydrosoluble (P2) ;
   → -III- au moins un plastifiant (PL) ;
   → -IV- et éventuellement au moins un agent tensioactif (TA) lubrifiant ;
   les composants P1, P2, PL de la pellicule d'enrobage satisfaisant aux caractéristiques suivantes :
   ⇒ fraction massique en poids sec de P1 par rapport à la masse totale de l'enrobage, comprise entre 40 et 90% et de préférence entre 50 et 80%;
   ⇒ fraction massique en poids sec P2/P1+P2 comprise entre 15 et 60 % et de préférence entre 15 et 55%;
   ⇒ fraction massique en poids sec PL/P1+PL comprise entre 1 et 30 % et de préférence entre 5 et 25 %.
- et cette pellicule d'enrobage représente au moins 3 % p/p sec, de préférence au moins 5% p/p sec de leur masse totale;
*à l'exclusion ou non des pellicules d'enrobage constituées par des compositions entériques et des pellicules d'enrobage de composition ci-après:*
*1 - au moins un polymère filmogène (P1) insoluble dans les liquides du tractus, présent à raison de 50 à 90, de préférence 50 à 80 % en poids sur sec par rapport à la masse totale de la composition d'enrobage et constitué par au moins un dérivé non hydrosoluble de la cellulose, à savoir l'éthylcellulose et*/*ou l'acétate de cellulose ;*
*2 - au moins un polymère azoté (P2) présent à raison de 2 à 25, de préférence 5 à 15* % *en poids sur sec par rapport à la masse totale de la composition d'enrobage et constitué par au moins un polyacrylamide et*/*ou un poly-N-vinylamide et*/*ou un poly-N-vinyl-lactame, à savoir le polyacrylamide et*/*ou la polyvinylpyrrolidone ;*
*3 - au moins un plastifiant présent à raison de 2 à 20, de préférence de 4 à 15 % en poids sur sec par rapport à la masse totale de la composition d'enrobage et constitué par au moins l'un des composés suivants : les esters du glycérol, les phtalates, les citrates, les sébaçates, les esters de l'alcool cétylique, l'huile de ricin, l'acide salicylique et la cutine ;*
*4 - et au moins un agent tensioactif et*/*ou lubrifiant, présent à raison de 2 à 20, de préférence de 4 à 15 % en poids sur sec par rapport à la masse totale de la composition d'enrobage et choisi parmi les tensioactifs anioniques, à savoir les sels alcalins ou alcalinoterreux des acides gras, l'acide stéarique et*/*ou oléique étant préférés, et*/*ou parmi les tensioactifs non ioniques, à savoir les esters de sorbitan polyoxyéthylénés et*/*ou les dérivés de l'huile de ricin polyoxyéthylénés, et*/*ou parmi les agents lubrifiants comme les stéarates de calcium, de magnésium, d'aluminium ou de zinc, ou comme le stéarylfumarate de sodium et*/*ou le béhénate de glycérol; ledit agent pouvant comprendre un seul ou un mélange des susdits produits ;*
pour fabriquer un médicament à base d'au moins un PA peu soluble et administrable par voie orale, avalable aisément et qui se libère in vivo de manière contrôlée, prolongée, et éventuellement retardée.

Selon encore un autre de ses objets, la présente invention concerne une méthode de traitement thérapeutique, dans laquelle on a recours à un médicament tel que défini ci-dessus en tant que produit *per se* ou en tant que produit obtenu par le procédé sus-décrit.

L'invention sera mieux comprise sur le plan de sa composition de ses propriétés et de son obtention, à la lecture des exemples ci-après, donnés uniquement à titre d'illustration et permettant de faire ressortir les variantes de réalisation et les avantages de l'invention.

### DESCRIPTION DES FIGURES :

- La Figure 1 représente la courbe du pourcentage de dissolution (% D) du principe actif PA, en fonction du temps (t) en heures (H), des microcapsules de l'exemple 1, dans le test de dissolution décrit dans les exemples qui suivent.
- La Figure 2 représente la courbe du pourcentage de dissolution (% D) du principe actif PA, en fonction du temps (t) en heures (H), des microcapsules de l'exemple 2, dans le test de dissolution décrit dans les exemples qui suivent.

### EXEMPLES

### Exemple 1

### Préparation de microcapsules d'Acyclovir :

### Etape 1 : Granulé

970 g d'Acyclovir et 30 g de Povidone (Plasdone® K29/32) sont préalablement mélangés à sec dans la cuve d'un granulateur à haut cisaillement (Lodige® M5MRK) pendant 5 minutes. Ce mélange pulvérulent est ensuite granulé à l'eau (200 g). Les granulés sont séchés à 40°C en étuve ventilée, puis calibrés sur grille de 500 µm. On sélectionne par tamisage la fraction 200-500 µm.

### Etape 2 : Enrobage

700 g de granulés obtenus précédemment sont enrobés dans un appareil à lit d'air fluidisé GLATT® GPCG1 par 50.65 g d'éthylcellulose (Ethocel® 7 Premium), 50.65 g de povidone (Plasdone® K29/32), 12.35 g de stéarate de Magnésium et 9.88 g d'huile de ricin dissous dans un mélange acétone / isopropanol (60/40 m/m),.

**Composition des microcapsules :**

| Ingrédient | % massique | Formule de fabrication (en g) |
|---|---|---|
| Granulés d'Acyclovir | **85.0** | 700.0L |
| - Plasdone® K29/32 | (2.55) | |
| - Acyclovir | (82.45) | |
| Enrobage | **15.0** | 123.5 |
| - Ethocel® 7 Premium | (6.15) | |
| - Plasdone® K29/32 | (6.15) | |
| - Stearate de Magnésium | (1.50) | |
| - Huile de ricin | (1.20) | |

### Test :

La cinétique de libération de l'Acyclovir est déterminée par un test de dissolution (Appareil de type II selon la pharmacopée Européenne 3è édition, milieu tampon phosphate pH 6.8, volume 900 ml, température 37°C, agitation palettes 100 tours/min, détection UV 268 nm).

### Résultat :

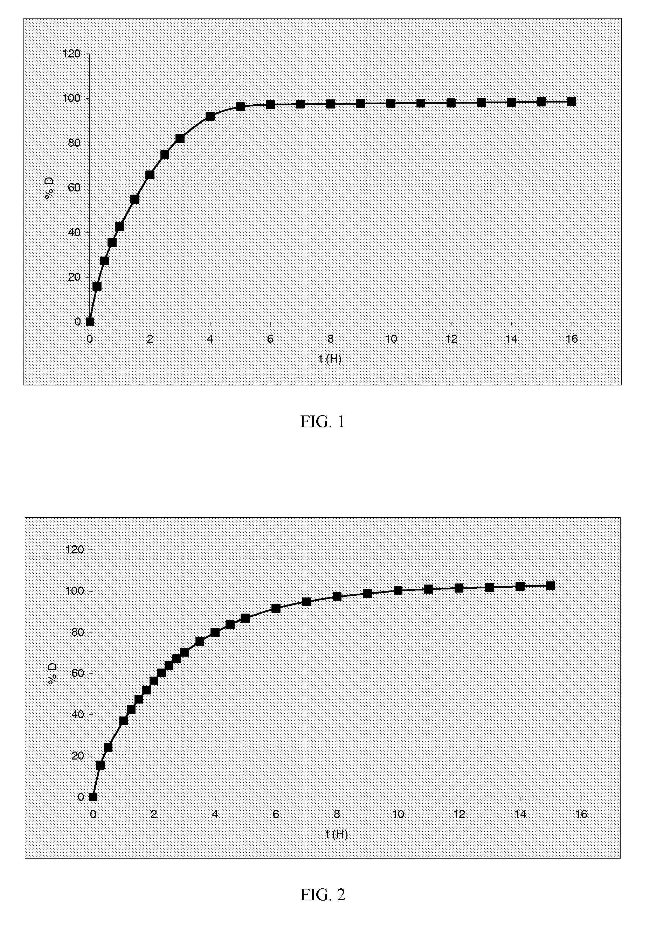

La Figure 1 ci-jointe montre le profil de dissolution obtenu par ces microcapsules.

La composition des microcapsules décrite ci-dessus permet d'obtenir un profil de dissolution caractérisé par 80% d'Acyclovir libéré à 3 heures.

### Exemple 2

### Préparation de microcapsules d'amoxicilline:

### Etape 1 : Granulé

970 g d'amoxicilline trihydrate et 30 g de Povidone (Plasdone® K29/32) ) sont préalablement mélangés à sec dans la cuve d'un granulateur à haut cisaillement (Lodige® M5MRK) pendant 5 minutes. Ce mélange pulvérulent est ensuite granulé à l'eau (200 g). Les granulés sont séchés à 40°C en étuve ventilée, puis calibrés sur grille de 500 µm. On sélectionne par tamisage la fraction 200-500 µm.

### Etape 2 : Enrobage

700 g de granulés obtenus précédemment sont enrobés dans un appareil à lit d'air fluidisé GLATT® GPCG1 par g d'éthylcellulose (Ethocel® 7 Premium), g de povidone (Plasdone® K29/32) et 0.96 g d'huile de ricin dissous dans un mélange acétone / isopropanol (60/40 m/m)

**Composition des microcapsules :**

| Ingrédient | % massique | Formule de fabrication (en g) |
|---|---|---|
| Granulés d'Amoxicilline | **82.0** | 700.0 |
| - Plasdone® K29/32 | (0.45) | |
| - Amoxicilline trihydrate | (14.55) | |
| Enrobage | **18.0** | 153.6 |
| - Ethocel® 7 Premium | (12.60) | |
| - Plasdone® K29/32 | (4.14) | |
| - Huile de ricin | (1.26) | |

### Test :

La cinétique de libération de l'amoxicilline est déterminée par un test de dissolution (Appareil de type II selon la pharmacopée Européenne 3è édition, milieu tampon phosphate pH 6.8, volume 900 ml, température 37°C, agitation palettes 100 tours/min, détection UV 240 nm).

### Résultat :

La Figure 2 ci-jointe montre le profil de dissolution obtenu pour ces microcapsules.

La composition des microcapsules décrite ci-dessus permet d'obtenir un profil de dissolution caractérisé par 80% d'amoxicilline libérée à 4 heures.

## Revendications

1. Microcapsules permettant la libération modifiée d'au moins un principe actif PA dont la solubilité dans l'eau est inférieure à 10 g/l à 25°C, *à l'exclusion des anti-hyperglycémiants,* pour la voie orale et du type de celles :
• constituées chacune par un coeur comportant au moins un principe actif et par une pellicule d'enrobage appliquée sur le coeur et régissant la libération modifiée du (ou des) PA,
• dont le diamètre moyen est inférieur à 1000 microns, de préférence compris entre 800 et 50 microns et plus préférentiellement encore compris entre 600 et 100 microns,
• dont la pellicule d'enrobage de chaque microcapsule contient les composants suivants :
→ -I-- au moins un polymère filmogène (P1) insoluble dans les liquides du tractus gastro-intestinal ;
→ -II-- au moins un polymère hydrosoluble (P2) ;
→ -III- au moins un plastifiant (PL) ;
• et ne contient pas d'agent tensioactif (TA) lubrifiant
**caractérisées**:
➢ en ce que leur pellicule d'enrobage représente 3 à 40% p/p sec de leur masse totale,
➢ et en ce que les composants P1, P2, PL de la pellicule d'enrobage satisfont aux caractéristiques suivantes :
➢ fraction massique en poids sec de P1 par rapport à la masse totale de l'enrobage, comprise entre 50 et 80%;
➢ fraction massique en poids sec P2/P1+P2 comprise entre 15 et 60 %;
➢ fraction massique en poids sec PL/P1+PL comprise entre 1 et 30 % ;
*à l'exclusion des microcapsules dont*
*- la pellicule d'enrobage est constituée par une composition entérique et de celles dont*
*- la pellicule d'enrobage a la composition ci-après:*
*1 - au moins un polymère filmogène (P1) insoluble dans les liquides du tractus, présent à raison de 50 à 90 % en poids sur sec par rapport à la masse totale de la composition d'enrobage et constitué par au moins un dérivé non hydrosoluble de la cellulose à savoir l'éthylcellulose et*/*ou l'acétate de cellulose ;*
*2 - au moins un polymère azoté (P2) présent à raison de 2 à 25 % en poids sur sec par rapport à la masse totale de la composition d'enrobage et constitué par au moins un polyacrylamide et*/*ou un poly-N-vinylamide et*/*ou un poly-N-vinyl-lactame, à savoir le polyacrylamide et*/*ou la polyvinylpyrrolidone;*
*3 - au moins un plastifiant présent à raison de 2 à 20 % en poids sur sec par rapport à la masse totale de la composition d'enrobage et constitué par au moins l'un des composés suivants : les esters du glycérol, les phtalates, les citrates, les sébacates, les esters de l'alcool cétylique, l'huile de ricin, l'acide salicylique et la cutine ;*
*4 - et au moins un agent tensioactif et*/*ou lubrifiant, présent à raison de 2 à 20 % en poids sur sec par rapport à la masse totale de la composition d'enrobage et choisi parmi les tensioactifs anioniques, à savoir les sels alcalins ou alcalinoterreux des acides gras, et*/*ou parmi les tensioactifs non ioniques, à savoir les esters de sorbitan polyoxyéthylénés et*/*ou les dérivés de l'huile de ricin polyoxyéthylénés, et*/*ou parmi les agents lubrifiants; ledit agent pouvant comprendre un seul ou un mélange des susdits produits .*

2. Microcapsules selon la revendication 1, **caractérisées en ce que** la fraction massique en poids sec P2/P1+P2 est comprise entre 15 et 55%.

3. Microcapsules selon la revendication 1, **caractérisées en ce que** la fraction massique en poids sec PL/P1+PL est comprise entre 5 et 25 %.

4. Microcapsules selon l'une quelconque des revendications 1 à 3, **caractérisées en ce que** P1 est sélectionné dans le groupe de produits suivants:
• les dérivés non hydrosolubles de la cellulose, de préférence l'éthylcellulose et/ou l'acétate de cellulose,
• les dérivés acryliques,
• les polyvinylacétates,
• et leurs mélanges.

5. Microcapsules selon l'une quelconque des revendications 1 à 4, **caractérisées en ce que** P2 est sélectionné dans le groupe de produits suivants:
• les dérivés hydrosolubles de la cellulose,
• les polyacrylamides,
• les poly-N-vinylamides,
• les poly-N-vinyl-lactames,
• les alcools polyvinyliques (APV),
• les polyoxyéthylènes (POE),
• les polyvinylpyrrolidones (PVP) (ces dernières étant préférées),
• et leurs mélanges.

6. Microcapsules selon l'une quelconque des revendications 1 à 5, **caractérisées en ce que** le plastifiant PL est sélectionné dans le groupe de produits suivants:
• le glycérol et ses esters, de préférence dans le sous-groupe suivant: glycérides acétylés, glycérolmonostéarate, glycéryltriacétate, glycéryltributyrate,
• les phtalates, de préférence dans le sous-groupe suivant : dibutylphthalate, diéthylphthalate, diméthylphthalate, dioctyl-phthalate,
• les citrates, de préférence dans le sous-groupe suivant : acétyltributylcitrate, acétyltriéthylcitrate, tributylcitrate, triéthyl-citrate,
• les sébacates, de préférence dans le sous-groupe suivant : diéthylsébacate, dibutylsébacate,
• les adipates,
• les azélates,
• les benzoates,
• les huiles végétales,
• les fumarates, de préférence le diéthylfumarate,
• les malates, de préférence le diéthylmalate,
• les oxalates, de préférence le diéthyloxalate,
• les succinates, de préférence le dibutylsuccinate,
• les butyrates,
• les esters de l'alcool cétylique,
• les malonates, de préférence le diéthylmalonate,
• l'huile de ricin (celle-ci étant particulièrement préférée),
• et leurs mélanges.

7. Médicament comprenant les microcapsules selon l'une quelconque des revendications 1 à 6.

8. Médicament selon la revendication 7, **caractérisé en ce qu'**il est sous forme solide de préférence : comprimé, gélule ou poudre, ou sous forme liquide, de préférence : suspension aqueuse.

9. Utilisation de microcapsules permettant la libération modifiée d'au moins un principe actif PA dont la solubilité dans l'eau est inférieure à 10 g/l à 25°C, *à l'exclusion des anti-hyperglycémiants,* pour la voie orale, pour fabriquer un médicament à base d'au moins un PA peu soluble et administrable par voie orale, avalable aisément et qui se libère in vivo de manière contrôlée, prolongée, et éventuellement retardée,.ces microcapsules présentant les caractéristiques suivantes :
• elles sont constituées chacune par un coeur comportant au moins un principe actif PA et par une pellicule d'enrobage appliquée sur le coeur et régissant la libération prolongée du (ou des) PA,
• leur diamètre moyen est inférieur à 1000 microns, de préférence compris entre 800 et 50 microns et plus préférentiellement encore compris entre 600 et 100 microns,
• la pellicule d'enrobage de chaque microcapsule contient les composants suivants :
→ -I-- au moins un polymère filmogène (P1) insoluble dans les liquides du tractus gastro-intestinal ;
→ -II-- au moins un polymère hydrosoluble (P2) ;
→ -III- au moins un plastifiant (PL) ;
• leur pellicule d'enrobage ne contient pas d'agent tensioactif (TA) lubrifiant
• leur pellicule d'enrobage représente 3 à 40% p/p sec de leur masse totale, et
• les composants P1, P2, PL de leur pellicule d'enrobage satisfont aux caractéristiques suivantes :
o fraction massique en poids sec de P1 par rapport à la masse totale de l'enrobage, comprise entre 50 et 80%;
o fraction massique en poids sec P2/P1+P2 comprise entre 15 et 60
%;
o fraction massique en poids sec PL/P1+PL comprise entre 1 et 30 % ;
*à l'exclusion de l'utilisation des microcapsules dont la pellicule d'enrobage est constituée par une composition entérique, et des microcapsules dont la pellicule d'enrobage a la composition ci-après:*
*1 - au moins un polymère filmogène (P1) insoluble dans les liquides du tractus, présent à raison de 50 à 90 % en poids sur sec par rapport à la masse totale de la composition d'enrobage et constitué par au moins un dérivé non hydrosoluble de la cellulose à savoir l'éthylcellulose et*/*ou l'acétate de cellulose ;*
*2 - au moins un polymère azoté (P2) présent à raison de 2 à 25 % en poids sur sec par rapport à la masse totale de la composition d'enrobage et constitué par au moins un polyacrylamide et*/*ou un poly-N-vinylamide et*/*ou un poly-N-vinyl-lactame, à savoir le polyacrylamide et*/*ou la polyvinylpyrrolidone;*
*3 - au moins un plastifiant présent à raison de 2 à 20 % en poids sur sec par rapport à la masse totale de la composition d'enrobage et constitué par au moins l'un des composés suivants : les esters du glycérol, les phtalates, les citrates, les sébacates, les esters de l'alcool cétylique, l'huile de ricin, l'acide salicylique et la cutine ;*
*4 - et au moins un agent tensioactif et*/*ou lubrifiant, présent à raison de 2 à 20 % en poids sur sec par rapport à la masse totale de la composition d'enrobage et choisi parmi les tensioactifs anioniques, à savoir les sels alcalins ou alcalinoterreux des acides gras, et*/*ou parmi les tensioactifs non ioniques, à savoir les esters de sorbitan polyoxyéthylénés et*/*ou les dérivés de l'huile de ricin polyoxyéthylénés, et*/*ou parmi les agents lubrifiants; ledit agent pouvant comprendre un seul ou un mélange des susdits produits.*

10. Utilisation d'une pellicule d'enrobage de microcapsules pour la voie orale permettant la libération modifiée d'au moins un principe actif PA dont la solubilité dans l'eau est inférieure à 10 g/l à 25°C, *à l'exclusion des anti-hyperglycémiants,* pour conférer auxdites microcapsules des propriétés de reproductibilité de la libération prolongée du (ou des) principe(s) actif(s),
ces microcapsules présentant les caractéristiques suivantes :
• elles sont constituées chacune par un coeur comportant au moins un principe actif et par une pellicule d'enrobage appliquée sur le coeur et régissant la libération prolongée du (ou des) principe(s) actif(s),
• leur diamètre moyen est inférieur à 1000 microns, de préférence compris entre 800 et 50 microns et plus préférentiellement encore compris entre 600 et 100 microns,
• dont la pellicule d'enrobage de chaque microcapsule contient les composants suivants :
→ -I-- au moins un polymère filmogène (P1) insoluble dans les liquides du tractus gastro-intestinal ;
→ -II-- au moins un polymère hydrosoluble (P2) ;
→ -III- au moins un plastifiant (PL) ;
➢ la pellicule d'enrobage représentant 3 à 40% p/p sec de leur masse totale,
➢ et les composants P1, P2, PL de la pellicule d'enrobage satisfaisant aux caractéristiques suivantes :
➢ fraction massique en poids sec de P1 par rapport à la masse totale de l'enrobage, comprise entre 50 et 80%;
➢ fraction massique en poids sec P2/P1+P2 comprise entre 15 et 60 %;
➢ fraction massique en poids sec PL/P1+PL comprise entre 1 et 30 % ;
*à l'exclusion de l'utilisation des microcapsules dont la pellicule d'enrobage est constituée par une composition entérique, et de celles dont- la pellicule d'enrobage a la composition ci-après:*
*1 - au moins un polymère filmogène (P1) insoluble dans les liquides du tractus, présent à raison de 50 à 90 % en poids sur sec par rapport à la masse totale de la composition d'enrobage et constitué par au moins un dérivé non hydrosoluble de la cellulose à savoir l'éthylcellulose et*/*ou l'acétate de cellulose ;*
*2 - au moins un polymère azoté (P2) présent à raison de 2 à 25 % en poids sur sec par rapport à la masse totale de la composition d'enrobage et constitué par au moins un polyacrylamide et*/*ou un poly-N-vinylamide et*/*ou un poly-N-vinyl-lactame, à savoir le polyacrylamide et*/*ou la polyvinylpyrrolidone;*
*3 - au moins un plastifiant présent à raison de 2 à 20 % en poids sur sec par rapport à la masse totale de la composition d'enrobage et constitué par au moins l'un des composés suivants : les esters du glycérol, les phtalates, les citrates, les sébacates, les esters de l'alcool cétylique, l'huile de ricin, l'acide salicylique et la cutine ;*
*4 - et au moins un agent tensioactif et*/*ou lubrifiant, présent à raison de 2 à 20 % en poids sur sec par rapport à la masse totale de la composition d'enrobage et choisi parmi les tensioactifs anioniques, à savoir les sels alcalins ou alcalinoterreux des acides gras, et*/*ou parmi les tensioactifs non ioniques, à savoir les esters de sorbitan polyoxyéthylénés et*/*ou les dérivés de l'huile de ricin polyoxyéthylénés, et*/*ou parmi les agents lubrifiants; ledit agent pouvant comprendre un seul ou un mélange des susdits produits* ,

11. Utilisation d'une pellicule d'enrobage de microcapsules selon la revendication 10 pour conférer auxdites microparticules des propriétés de reproductibilité de la libération prolongée du (des) principe(s) actif(s) dans le test de dissolution réalisé avec un appareil de type II selon la Pharmacopée européenne 3è édition, en milieu tampon phosphate pH 6.8, volume 900 ml, température 37°C, agitation des palettes 100 tours/min.

12. Utilisation selon la revendication 10 ou 11 **caractérisée en ce que** la pellicule d'enrobage contient en outre un agent tensioactif (TA) lubrifiant.
